(19) Europäisches Patentamt
European Patent Office
Office européen des brevets

(11) EP 2 405 911 B1

(12) **EUROPEAN PATENT SPECIFICATION**

(45) Date of publication and mention of the grant of the patent:
**06.03.2013 Bulletin 2013/10**

(21) Application number: **10709040.9**

(22) Date of filing: **10.03.2010**

(51) Int Cl.:
*A61K 31/4025* (2006.01) *A61P 9/00* (2006.01)
*A61P 9/10* (2006.01) *A61P 3/06* (2006.01)
*A61K 31/4439* (2006.01)

(86) International application number:
**PCT/GB2010/050408**

(87) International publication number:
**WO 2010/103319 (16.09.2010 Gazette 2010/37)**

(54) **USE OF ATORVASTATIN LACTOLS AS MEDICAMENTS**

VERWENDUNG VON ATORVASTATIN-LAKTOLEN ALS MEDIKAMENTE

UTILISATION DE LACTOLES D'ATORVASTATINE EN TANT QUE MÉDICAMENTS

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **10.03.2009 GB 0904102**

(43) Date of publication of application:
**18.01.2012 Bulletin 2012/03**

(73) Proprietor: **Redx Pharma Limited**
**Liverpool L69 7ZD (GB)**

(72) Inventors:
- **LINDSAY, Derek**
  **Wigan WN1 2TB (GB)**
- **JACKSON, Peter**
  **Wigan WN1 2TB (GB)**

(74) Representative: **Harrison Goddard Foote**
**Belgrave Hall**
**Belgrave Street**
**Leeds**
**LS2 8DD (GB)**

(56) References cited:
**EP-A1- 1 834 944** **WO-A1-2005/012246**
**WO-A2-2005/092867** **WO-A2-2006/122644**

- **TATAROV: "Synthesis of the chiral side of chain of statins" EUROPEAN JOURNAL OF ORGANIC CHEMIOSTRY, 2006, pages 5543-5550, XP8121532**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 405 911 B1

Printed by Jouve, 75001 PARIS (FR)

## Description

The present invention relates to atorvastatin lactols. In particular, the present invention relates to the use of atorvastatin lactols in the manufacture of a medicament for treating certain conditions. Conditions that are treatable using the compounds of the present invention include conditions which are modulated by the enzyme 3-hydroxy-3-methylglutaryl-coenzyme A reductase (HMG-CoA reductase). Inhibition of the enzyme therefore represents a viable therapy for a number of diseases. The compounds used in the invention are 6-(3- or 4-carboxamido-substituted pyrrol-1-yl)-4-hyroxy-3,5-dihydropyran-2-ol derivatives.

Trans-6-[2-(3- or 4- carboxamido-substituted pyrrol-1-yl)alkyl]-4-hydroxypyran-2-one compounds are lactones which were first disclosed in US 4,681,893. This document also disclosed their corresponding ring opened acid equivalents. The lactones apparently do not have intrinsic activity of their own. However, the corresponding ring-opened acid equivalents are useful as cholesterol biosynthesis inhibitors. Also disclosed in US 4,681,893 are various methods of manufacture for such compounds.

Atorvastatin, which is the *R* form of the ring-opened acid of trans-5-(4-fluorophenyl)-2-(1-methylethyl)-N,4-diphenyl-1-[2-tetrahydro-4-hydroxy-6-oxo-2H-pyran-2-yl)ethyl]-1H-pyrrole-3-carboxamide, and its use in the inhibition of the biosynthesis of cholesterol was first disclosed in EP 0409281. Atorvastatin both in racemic form, and in the form of its [R-(R*,R*)] isomer is a potent inhibitor of HMG-CoA enzyme.

Clin Invest Med, Volume 24, No 5, p258-72, 2001 (Baker and Tamopolsky) discloses that whilst statins having an open, hydroxy acid conformation are active, the lactone, closed-ring analogue is inactive. Hepatic hydrolysis at alkaline pH decyclises and hence activates the lactone prodrugs lovastatin and simvastatin in vivo. However, one problem with such compounds is that extensive first path metabolism leads to rapid clearance of these statins.

Similarly, Trends in Pharmacological Sciences, Volume 19, Issue 1, 1 January 1998, Pages 26-37 discloses that the inactive lactones must be metabolised to their corresponding open hydroxy acid forms in order to inhibit HMG-CoA reductase.

The lactone form, and also the ring opened active form, may suffer problems in terms of stability over an extended period of time. This represents a significant problem during manufacture of an active principal or during extended storage of the same in a pharmacy. For example, loss of the hydroxy group in a dehydration reaction may occur. The resulting decomposition product may have a double bond that is conjugated with the lactone carbonyl group and this may tend to favour formation of the decomposition product. Equally, in the ring opened form, one of the possible decomposition products could also have a conjugated double bond with the acid carbonyl group.

It is therefore an aim of the present invention to provide compounds capable of inhibiting HMG-CoA reductase. Atorvastatin is a very potent inhibitor of HMG-CoA reductase. It is also therefore an aim of the present invention to provide compounds capable of inhibiting HMG-CoA reductase which have an IC50 value comparable to or better than that of atorvastatin. Ideally, these compounds will have good stability and bioavailability relative to atorvastatin. It is thus an aim to provide compounds having improved stability. Ideally, the compounds will have an extended shelf-life. It is thus an aim of the present invention to provide compounds capable of inhibiting HMG-CoA reductase which have increased half-life. It is thus an aim of the present invention to provide further compounds capable of inhibiting HMG-CoA reductase and having improved bioavailability. It is also an aim of the present invention to provide compounds capable of inhibiting HMG-CoA reductase and increasing promotion of high density lipoprotein (HDL). It is also an aim of the present invention to provide compounds capable of reducing low density lipoprotein (LDL) and increasing promotion of high density lipoprotein (HDL). Specifically, it is an aim of the present invention to provide compounds capable of reducing low density lipoprotein (LDL) and increasing promotion of high density lipoprotein (HDL) by more than 10%, preferably up to 15% or higher. The invention thus seeks to provide therapies for inhibiting cholesterol biosynthesis. The invention also aims to treat a range of diseases in which cholesterol formation is inhibited.

This invention provides compounds that achieve one or more of the above aims.

According to one aspect, the present invention provides a use of a compound of Formula I and pharmaceutically acceptable salts and solvates thereof:

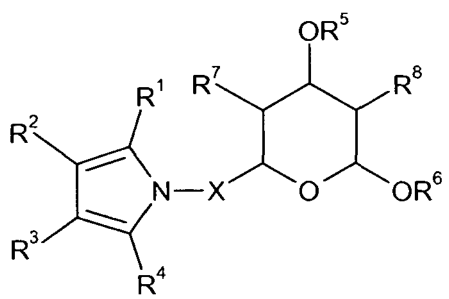

(I)

in the manufacture of a medicament for treating a condition which is modulated the enzyme 3-hydroxy-3-methylglutaryl-coenzyme A reductase (HMG-CoA reductase) according to the claims, wherein:

$R^1$, $R^4$ and one of $R^2$ and $R^3$ are independently selected from the group consisting of: hydrogen, halo, $C_{1-6}$ alkyl, $C_{2-6}$ alkenyl, $C_{3-6}$ cycloalkyl, aryl, $C_{1-4}$ alkyl aryl, heterocyclyl, and $C_{1-4}$ alkyl heteroaryl;

the other of $R^2$ and $R^3$ is $-CONR^9R^{10}$ where $R^9$ and $R^{10}$ are independently selected from the group consisting of: hydrogen, $C_{1-6}$ alkyl, $C_{2-6}$ alkenyl, aryl, $C_{1-4}$ alkyl aryl, heteroaryl, $C_{1-4}$ heteroaryl;

$R^5$ and $R^6$ are independently selected from the group consisting of: hydrogen, $C_{1-6}$ alkyl, $C_{1-6}$ haloalkyl, $C_{2-6}$ alkenyl, $C_{3-6}$ cycloalkyl, aryl, $C_{1-6}$ alkyl aryl, $C_{1-6}$ alkanoyl aryl, heteroaryl, $C_{1-6}$ alkanoyl heteroaryl, and $C_{1-6}$ alkyl heteroaryl; provided always that both $R^5$ and $R^6$ are not hydrogen;

$R^7$ and $R^8$ are independently selected from the group consisting of: H, $C_{1-4}$ alkyl and halo;

X is $-(CR^aR^b)_m(CR^a{=}CR^b)_n(CR^aR^b)_o$ where $R^a$ and $R^b$ are independently selected from the group consisting of: H, methyl, ethyl and halo and m, n, and o are independently 0, 1, 2, or 3 provided that m + n + o is not more than 3; and wherein

each of the above groups $R^1$ to $R^{10}$ may, where chemically possible, be independently optionally substituted by from 1 to 5 groups chosen independently at each occurrence from the groups consisting of: halo, $C_{1-3}$ alkyl, halo $C_{1-3}$ alkyl, $C_{1-3}$ alkoxy, $C_{1-3}$ haloalkoxy, hydroxy, and cyano.

Usually conditions that are modulated by HMG-CoA reductase are conditions according to the claims that would be treated by the inhibition of the enzyme using a compound of the present invention.

According to another aspect, the present invention provides a compound of Formula I and pharmaceutically acceptable salts and solvates thereof:

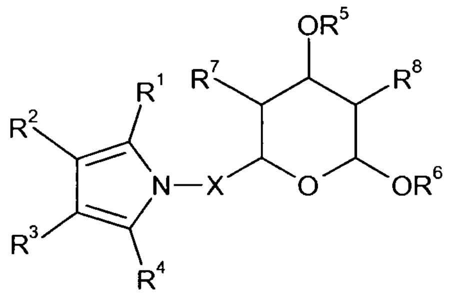

(I)

for use in treating a condition treatable by the inhibition of the enzyme 3-hydroxy-3-methylglutaryl-coenzyme A reductase (HMG-CoA reductase) according to the claims wherein $R^1$ - $R^{10}$, $R^a$, $R^b$, X, m, n and o are as defined above.

Compounds have activity in their own right or may in certain cases ring open under physiological conditions to corresponding compounds having inhibitory activity.

Pharmaceutically acceptable salts of the compounds of formula (1) include the acid addition and base salts thereof.

Suitable acid addition salts are formed from acids which form non-toxic salts. Examples include the acetate, aspartate, benzoate, besylate, bicarbonate/carbonate, bisulphate/sulphate, borate, camsylate, citrate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methylsulphate, naphthylate, 1 ,5-naphthalenedisulfonate, 2-napsylate, nicotinate, nitrate, orotate, oxalate, palmitate, pamoate, phosphate/hydrogen phosphate/dihydrogen phosphate, saccharate, stearate, succinate, tartrate, tosylate and trifluoroacetate salts.

Suitable base salts are formed from bases which form non-toxic salts. Examples include the aluminium, arginine, benzathine, calcium, choline, diethylamine, diolamine, glycine, lysine, magnesium, meglumine, olamine, potassium, sodium, tromethamine and zinc salts. Hemisalts of acids and bases may also be formed, for example, hemisulphate and hemicalcium salts. For a review on suitable salts, see "Handbook of Pharmaceutical Salts: Properties, Selection, and Use" by Stahl and Wermuth (Wiley-VCH, Weinheim, Germany, 2002).

Pharmaceutically acceptable salts of compounds of formula (1) may be prepared by one or more of three methods:

(i) by reacting the compound of formula (1) with the desired acid or base;
(ii) by removing an acid- or base-labile protecting group from a suitable precursor of the compound of formula (1) or by ring-opening a suitable cyclic precursor, for example, a lactone or lactam, using the desired acid or base; or
(iii) by converting one salt of the compound of formula (1) to another by reaction with an appropriate acid or base or by means of a suitable ion exchange column.

All three reactions are typically carried out in solution. The resulting salt may precipitate out and be collected by filtration or may be recovered by evaporation of the solvent. The degree of ionisation in the resulting salt may vary from completely ionised to almost non-ionised.
The compounds of the invention may exist in both unsolvated and solvated forms. The term 'solvate' is used herein to describe a molecular complex comprising the compound of the invention and a stoichiometric amount of one or more pharmaceutically acceptable solvent molecules, for example, ethanol. The term 'hydrate' is employed when said solvent is water.
Included within the scope of the invention are complexes such as clathrates, drug-host inclusion complexes wherein, in contrast to the aforementioned solvates, the drug and host are present in stoichiometric or non-stoichiometric amounts. Also included are complexes of the drug containing two or more organic and/or inorganic components which may be in stoichiometric or non-stoichiometric amounts. The resulting complexes may be ionised, partially ionised, or non- ionised. For a review of such complexes, see J Pharm Sci, 64 (8), 1269-1288 by Haleblian (August 1975).
Hereinafter all references to compounds of formula (1) include references to salts, solvates and complexes thereof and to solvates and complexes of salts thereof.
The compounds of the invention include compounds of formula (1) as hereinbefore defined, including all polymorphs and crystal habits thereof, optical, geometric and tautomeric isomers as hereinafter defined. Isotopically-labeled compounds of formula (1) are also presently disclosed.
Before purification, the compounds of the present invention may exist as a mixture of enantiomers depending on the synthetic procedure used. For example, the compounds of the present invention may exist as a mixture of enantiomers having a ratio of between 2:1 and 3:1, though they may also occur in other ratios. The enantiomers can be separated by conventional techniques known in the art. Thus the invention covers individual enantiomers as well as mixtures thereof. When the chemical structures disclosed herein includes an '*', it is intended that the compound is a mixture of enantiomers having a ratio of between 2:1 and 3:1.
For some of the steps of the process of preparation of the compounds of formula (1), it may be necessary to protect potential reactive functions that are not wished to react, and to cleave said protecting groups in consequence. In such a case, any compatible protecting radical can be used. In particular methods of protection and deprotection such as those described by T.W. GREENE (Protective Groups in Organic Synthesis, A. Wiley- Interscience Publication, 1981) or by P. J. Kocienski (Protecting groups, Georg Thieme Verlag, 1994), can be used. All of the above reactions and the preparations of novel starting materials used in the preceding methods are conventional and appropriate reagents and reaction conditions for their performance or preparation as well as procedures for isolating the desired products will be well-known to those skilled in the art with reference to literature precedents and the examples and preparations hereto.
Also, the compounds of formula (1) as well as intermediate for the preparation thereof can be purified according to various well-known methods, such as for example crystallization or chromatography.
In an embodiment, R is selected from the group consisting of: hydrogen, $C_{1-6}$ alkyl, $C_{2-6}$ alkenyl or $C_{3-6}$ cycloalkyl. In an embodiment, $R^1$ is selected from the group consisting of: $C_{2-6}$ alkenyl or $C_{3-6}$ cycloalkyl. In an alternative embodiment, $R^1$ is $C_{1-6}$ alkyl. In an embodiment, $R^1$ is methyl, ethyl, propyl or butyl. In an embodiment, $R^1$ is i-propyl.
In an embodiment, $R^2$ is $-CONR^9R^{10}$.
In an embodiment, $R^3$ is selected from the group consisting of: aryl, $C_{1-4}$ alkyl aryl, heteroaryl and $C_{1-4}$ alkyl heteroaryl. In an embodiment, $R^3$ is selected from the group consisting of: aryl and $C_{1-4}$ alkyl aryl. In an embodiment, $R^3$ is aryl. In an embodiment, $R^3$ is phenyl.
In an embodiment, $R^4$ is selected from the group consisting of: aryl, $C_{1-4}$ alkyl aryl, heteroaryl and $C_{1-4}$ alkyl heteroaryl, wherein each of the aforementioned groups may be optionally substituted as discussed above in relation to the first aspect. In an embodiment, $R^4$ is selected from the group consisting of: aryl and $C_{1-4}$ alkyl aryl. In an embodiment, $R^4$ is aryl. In an embodiment, $R^4$ is phenyl. In an embodiment, $R^4$ is substituted with halo, optionally wherein the halo is fluorine. In an embodiment, $R^4$ is 4-fluorophenyl.
In an embodiment, $R^5$ is selected from the group consisting of: hydrogen, $C_{1-6}$ alkyl, aryl, $C_{1-4}$ alkyl aryl, heteroaryl and $C_{1-4}$ alkyl heteroaryl. In an embodiment, $R^5$ is selected from the group consisting of: hydrogen, $C_{1-4}$ alkyl aryl and $C_{1-4}$ alkyl heteroaryl. In an embodiment, $R^5$ is $C_{1-6}$ alkanoyl heteroaryl, e.g. methanoyl heteroaryl. In a preferred embodiment, $R^5$ is methanoyl pyridyl, e.g. 2-methanolyl pyridine, 3-methanolyl pyridine or 4-methanolyl pyridine, preferably 3-methanolyl pyridine. In an embodiment, $R^5$ is hydrogen. In an alternative embodiment, $R^5$ is $C_{1-4}$ alkyl aryl, e.g. $-C_1$ alkyl-Ph, $-C_2$ alkyl-Ph, $-C_3$ alkyl-Ph, or $-C_4$ alkyl-Ph. In an embodiment, $R^5$ is benzyl.
In an embodiment, $R^6$ is the same as $R^5$.

In a further embodiment, $R^6$ is selected from the group consisting of: $C_{1-6}$ alkyl, $C_{2-6}$ alkenyl, $C_{3-6}$ cycloalkyl and aryl. In a preferred embodiment, $R^6$ is $C_{1-6}$ alkyl. In further preferred embodiment, $R^6$ is methyl, ethyl, n-propyl, i-propyl or t-butyl. In another preferred embodiment, $R^6$ is $C_{2-6}$ alkenyl. In another preferred embodiment, $R^6$ is allyl. In another preferred embodiment, $R^6$ is $C_{3-6}$ cycloalkyl. In another preferred embodiment, $R^6$ is cyclohexyl. In another embodiment, $R^6$ is aryl, such as optionally substituted phenyl.

In an embodiment, $R^7$ is H.

In an embodiment, $R^8$ is H.

In an embodiment, $R^9$ is hydrogen or $C_{1-4}$ alkyl. In an embodiment, $R^9$ is hydrogen.

In an embodiment, $R^{10}$ is selected from the group consisting of: aryl, $C_{1-4}$ alkyl aryl, heteroaryl and $C_{1-4}$ alkyl heteroaryl. In an embodiment, $R^{10}$ is selected from the group consisting of: aryl and $C_{1-4}$ alkyl aryl. In an alternative embodiment, $R^{10}$ is selected from the group comprising: heteroaryl and $C_{1-4}$ alkyl heteroaryl. In an embodiment, $R^{10}$ is phenyl.

In an embodiment, n is 0. In an embodiment, m = 1, n = 0 and o = 1 or m = 2, n = 0 and o = 0 or m = 0, n = 0 and o = 2. In an alternative embodiment, m = 3, n = 0 and o = 0. In an alternative embodiment, m = 1, n = 0 and o = 0. in an alternative embodiment, m = 1, n = 1 and o = 0, or m = 0, n = 1 and 0 = 1.

In an embodiment, $R^a$ is H at each occurrence.

In an embodiment, $R^b$ is H at each occurrence.

In a further embodiment, each $R^a$ is H, each $R^b$ is H and m = 2, n = 0 and o = 0.

Aryl groups include aromatic ring systems comprising 6, 7, 8, 9, 10, 11, 12, 13, 14, 15 or 16 ring carbon atoms. Aryl groups may consist of a single ring but may include a polycyclic ring system, having two or more rings, at least one of which is aromatic. Aryl groups include: phenyl, naphthyl, fluorenyl, azulenyl, indenyl and anthryl groups.

In an embodiment, the aryl group is phenyl.

Heteroaryl groups include aromatic heterocyclic ring systems having 5, 6, 7, 8, 9, 10, 11, 12, 13, 14, 15 or 16 ring atoms with 1 to 4 heteroatoms independently selected from nitrogen, oxygen and sulfur. The group may be a polycyclic ring system, having two or more rings, at least one of which is aromatic, but is more often monocyclic. Preferred heteroaryl groups are monocyclic groups containing 5 or 6 ring atoms. Heteroaryl groups include: pyrrolyl, pyrazolyl, imidazolyl, pyrazinyl, oxazolyl, isoxazolyl, thiazolyl, furyl, thiophenyl, pyridyl, pyrimidyl, benzimidazolyl, indolyl, isoquinolyl, quinoxalinyl and quinolyl.

In an embodiment, the heteroaryl group is selected from the group consisting of: pyridine, pyrimidine, pyrazine, pyrazole, and oxazole. Preferably the heteroaryl group is pyridine.

When one or more of the above groups is optionally substituted, each optional substituent is preferably an independently chosen halo atom. Amongst halo, chloro and fluoro are preferred. Preferably, the halo atoms are the same when there are more than one.

In an embodiment, $R^1$ is $C_{1-4}$alkyl, preferably i-propyl, and $R^4$ is optionally substituted aryl, preferably 4-fluorophenyl.

In another embodiment, $R^2$ is -CONR$^9$R$^{10}$ wherein $R^9$ is optionally substituted aryl, preferably phenyl; $R^{10}$ is hydrogen; and $R^3$ is optionally substituted aryl, preferably phenyl.

In a further embodiment, $R^1$ is $C_{1-4}$alkyl, preferably i-propyl; $R^2$ is -CONR$^9$R$^{10}$ wherein $R^9$ is optionally substituted aryl, preferably phenyl, $R^{10}$ is hydrogen; $R^3$ is optionally substituted aryl, preferably phenyl; and $R^4$ is optionally substituted aryl, preferably 4-fluorophenyl.

The relationship between the groups $R^5$ and $R^6$ is important for the activity of the compounds. Thus both $R^5$ and $R^6$ cannot be hydrogen. In one embodiment, $R^5$ is not hydrogen. In one embodiment, $R^6$ is not hydrogen.

In another embodiment, $R^5$ is optionally substituted benzyl and $R^6$ is optionally substituted $C_{1-6}$ alkyl. In this embodiment, preferably the alkyl group is propyl or butyl, preferably isopropyl or tertbutyl. In another embodiment, $R^5$ is optionally substituted benzyl and $R^6$ is optionally substituted $C_{2-6}$ alkenyl. In this embodiment, preferably the alkenyl group is allyl.

In another embodiment, $R^5$ is H and $R^6$ is optionally substituted $C_{1-6}$ alkyl. In this embodiment, preferably the alkyl group is propyl. In another embodiment, $R^5$ is H and $R^6$ is $C_{3-6}$ cycloalkyl. In this embodiment, preferably the cycloalkyl group is cyclohexyl. In another embodiment, $R^5$ is H and $R^6$ is optionally substituted aryl. In this embodiment, preferably the optionally substituted aryl is optionally substituted phenyl, e.g. phenyl substituted by alkoxy (e.g. methoxy).

In another embodiment, $R^5$ is $C_{1-6}$ alkanoyl heteroaryl and $R^6$ is optionally substituted $C_{1-6}$ alkyl. In this embodiment, the alkyl group is preferably methyl and the $C_{1-6}$ alkanoyl heteroaryl group is preferably methanoyl heteroaryl, (e.g. methanoyl pyridine).

In an embodiment, the compound has a structure selected from:

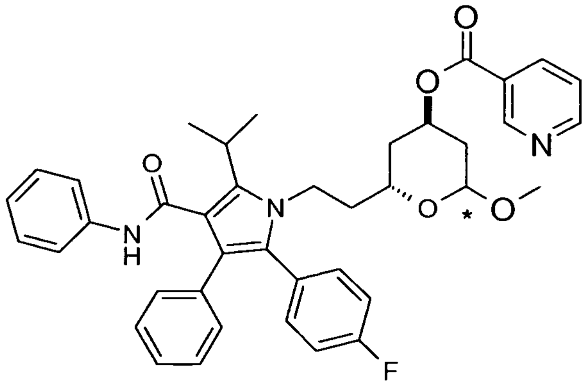

;

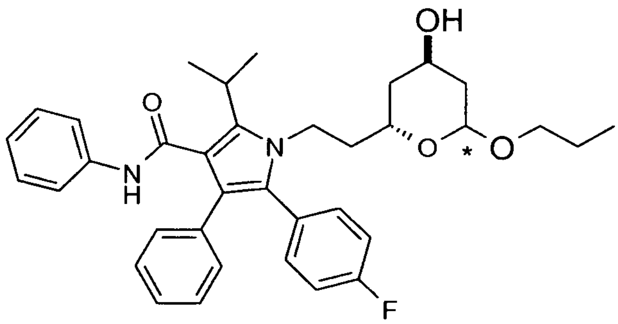

;

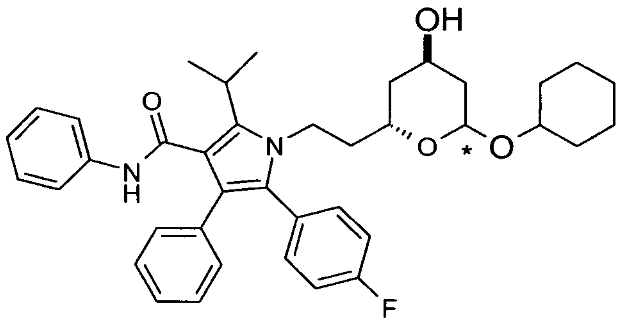

;

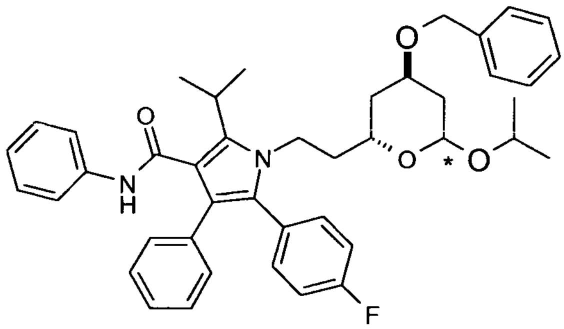

;

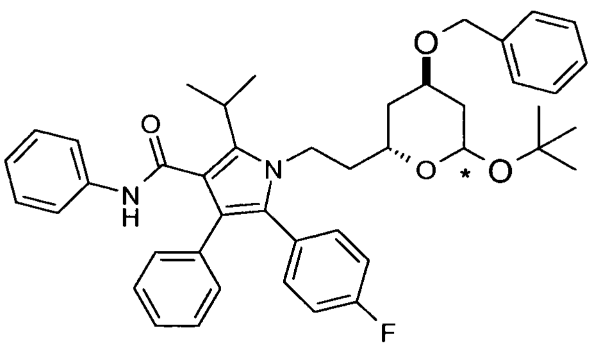

;

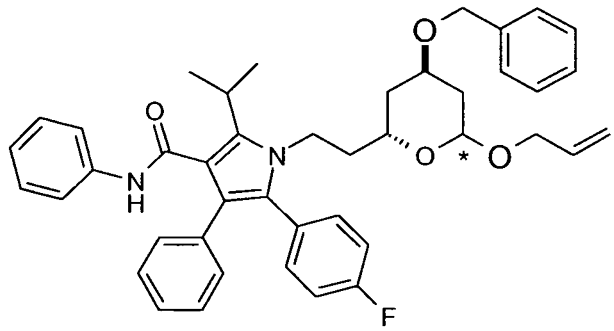

;

and

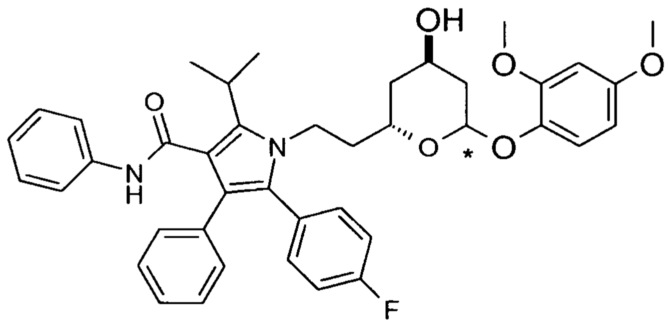

As mentioned above, statins having an open, hydroxy acid conformation are known to have an inhibitory effect on HMG-CoA reductase. It is also known that the lactone, closed-ring analogue of such hydroxy acids are inactive with respect to inhibiting HMG-CoA reductase and that decyclisation of the lactone is necessary to activate the lactone. However, we have found that functionalised lactols of the present invention have a significant inhibitory effect on HMG-CoA reductase in their own right. This is surprising in view of the fact that these molecules are conformationally constrained in ring closed form.

Examples of conditions that may be treated by the inhibition of HMG-CoA reductase include hypercholesterolemia, atherosclerosis and hyperlipidemia. Statins have been used in the secondary prevention of cardiovascular disease, or in the primary prevention of cardiovascular disease when the risk for cardiovascular disease is significantly raised. It is therefore expected that the compound of the present invention will have utility in the treatment or prevention of cardiovascular diseases due to their inhibitory activity. Example cardiovascular diseases which may be treatable by the compounds of the present invention include: coronary heart disease, myocardial infarction, stroke and peripheral artery disease. In addition, these compounds may also have a beneficial effect in the treatment of inflammation, dementia, cancer, nuclear cataracts, diabetes and hypertension.

The conditions that may be treated by the inhibition of HMG-CoA reductase may be a condition of the human or animal body. These compounds are intended in particular for human patients.

The atorvastatin derivatives of the present invention can be assayed using the following procedure in which the plasma triglyceride level is measured after treating a rat with a compound of the present invention (or atorvastatin).

The change in rat plasma triglyceride levels is considered to be a fair test for determining HMG CoA reductase activity.

The procedure used is as follows: male SD rats (Harlan) are housed in groups of 6 under a 12h light dark cycle (lights on 07.00 h) with free access to food (normal laboratory chow) and water. Animals between 148-183 g are allocated to treatment groups of 8 balanced by body weight and treatments are balanced across cages.

Solutions including 5 mg/mL of the atorvastatin analogues (in e.g. 10% PEG300/10% cremophor/80% methyl cellulose (0.5%)) and a suspension including 5mg/kg of atorvastatin (formulated in 0.5% Tween in 0.5% methyl cellulose) are made.

The rat subjects are orally dosed with one of the atorvastatin analogues (25mg/kg) or atorvastatin (25 mg/kg po), BID for 3 or 5 days.

Sixteen hours after the last treatment, terminal plasma samples are taken, stored at - 20°C, and transported on dry ice for analysis of triglyceride levels.

Data for each time-point are analysed by 1-way ANOVA and post-hoc Dunnett's test.

Processes for the manufacture of the compounds of the present invention are disclosed in WO2005/012246, in particular, in the examples.

The present disclosure also includes the synthesis of all pharmaceutically acceptable isotopically-labelled compounds of formula (I) wherein one or more atoms are replaced by atoms having the same atomic number, but an atomic mass or mass number different from the atomic mass or mass number usually found in nature.

Examples of isotopes suitable for inclusion in the compounds of the invention include isotopes of hydrogen, such as $^{2}H$ and $^{3}H$, carbon, such as $^{11}C$, $^{13}C$ and $^{14}C$ , chlorine, such as $^{36}Cl$, fluorine, such as $^{18}F$, iodine, such as $^{123}I$ and $^{125}I$, nitrogen, such as $^{13}N$ and $^{15}N$, oxygen, such as $^{15}O$, $^{17}O$ and $^{18}O$, phosphorus, such as $^{32}P$, and sulphur, such as $^{35}S$.

Certain isotopically-labelled compounds, for example, those incorporating a radioactive isotope, are useful in drug and/or substrate tissue distribution studies. The radioactive isotopes tritium, i.e. $^{3}H$, and carbon-14, i.e. $^{14}C$, are particularly useful for this purpose in view of their ease of incorporation and ready means of detection.

Substitution with heavier isotopes such as deuterium, i.e. $^{2}H$, may afford certain therapeutic advantages resulting from greater metabolic stability, for example, increased *in vivo* half-life or reduced dosage requirements, and hence may be preferred in some circumstances.

Substitution with positron emitting isotopes, such as $^{11}C$, $^{18}F$, $^{15}O$ and $^{13}N$, can be useful in Positron Emission Topography (PET) studies for examining substrate receptor occupancy.

Isotopically-labelled compounds can generally be prepared by conventional techniques known to those skilled in the art or by processes analogous to those described using an appropriate isotopically-labelled reagent in place of the non-labelled reagent previously employed.

Throughout the description and claims of this specification, the words "comprise" and "contain" and variations of the words, for example "comprising" and "comprises", means "including but not limited to", and is not intended to (and does not) exclude other moieties, additives, components, integers or steps.

Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, compounds, chemical moieties or groups described in conjunction with a particular aspect, embodiment or example of the invention are to be understood to be applicable to any other aspect, embodiment or example described herein unless incompatible therewith.

## General Procedure

All assays were carried out in a reaction buffer containing 100nM $K_xPO_4$ at pH 7.2, 1 mM EDTA, 500mM KCl and 1 mg/ml BSA. The concentrations of NADPH and HMG-CoA were both 200$\mu$M. The enzyme concentration used is unknown although this concentration is 10-fold lower than that of the stock solution purchased. Inhibitors were dissolved in 75% DMSO. Where inhibitors were found to be insoluble or only partly soluble in 75% DMSO, 100% DMSO was used. Reactions were activated by the addition of enzyme and agitated for 12 seconds following the addition. Absorbance readings were then taken every 20 seconds for 600 seconds. In initial tests the concentration of each inhibitor was set at 50nM to identify which compounds were the better inhibitors, compared to the known Pravastatin inhibitor. After these were identified, assays were carried out varying their concentrations from 0nM to 50nM allowing IC50 values to be calculated.

## Example 1

The following procedure was followed using a HMG-CoA Reductase assay kit obtained from Sigma-Aldrich (catalogue number CS1090). The assay is based on the spectrophotometric measurement of the decrease in absorbance

at 340 nm of NADPH in solution. A decrease in absorbance is caused by the oxidation of NADPH by the catalytic subunit of HMGR in the presence of the substrate HMG-CoA. Effective inhibition of the HMG-CoA leads to a reduction in oxidation of NADPH which in turn leads to a smaller reduction in the absorbance at 340 nm over time. This is illustrated in the following reaction scheme:

$$HMG\text{-}CoA + 2NADPH + 2H^+ \rightarrow mevalonate + 2NADP^+ + CoA\text{-}SH$$

Compounds showing the best inhibitory action are those which reduce the absorbance least.

**Preparation of the assay solution**

Ultrapure water (17 MΩ-cm or equivalent was used for the preparation of reagents and throughout the procedure.
First, an assay buffer solution was prepared using the following method: 0.2 ml of assay buffer, 5 x (catalogue number A5981) was diluted with 0.8 ml of ultrapure water. The resulting buffer solution was kept on ice or stored at -20°C for further use.
Next, 25 mg of NADPH (catalogue number N6505) was reconstituted with 1.5 ml of the buffer solution. The reconstituted NADPH was stored in working aliquots at -20°C.
The HMG-CoA substrate solution (catalogue number S7447), HMG-CoA reductase (catalogue number H8789) and inhibitor solution (e.g. pravastatin, catalogue number I5909) were kept on ice throughout the procedure.
1. Before beginning, the spectrophotometer was set at 37°C and 340 nm, with a kinetic programme: 1 ml sample, read every 20 seconds for up to 10 minutes.
2. The appropriate volumes of the reaction solutions were added according to Table 1 (1 ml assay).

**Table 1**

| Reaction volumes for 1 ml samples | | | | | |
|---|---|---|---|---|---|
| **Sample** | **1x Assay buffer** | **Test compound / Pravastatin** | **NADPH** | **HMG-CoA** | **HGMG** |
| Blank | 920 μl | - | 20 μl | 60 μl | - |
| Activity | 915 μl | - | 20 μl | 60 μl | 5 μl |
| Inhibition | 910 μl | 5 μl | 20 μl | 60 μl | 5 μl |

The reagents were added to the reaction in the following order:

a. Add a buffer to all samples.
b. Add the inhibitor (test compound/Pravastatin) to the inhibition sample.
c. Add the reconstituted NADPH to all samples.
d. Add Substrate Solution (HMG-CoA) to all samples.
e. Add HMG-CoA Reductase (HMGR) to the Activity and Inhibition samples.
f. Mix the samples thoroughly.

3. The kinetics programme was started immediately. The activity of the product was calculated according to the following equation:

$$\text{Units/mgP} = \frac{(\Delta A_{340}/\text{min}_{\text{sample}} - \Delta A_{340}/\text{min}_{\text{control}}) \times TV}{12.44 \times V \times 0.6 \times LP}$$

where:

12.44 = €$^{mM}$ - the extinction coefficient for NADPH at 340 nm is 6.22 $mM^{-1}cm^{-1}$. 12.44 represents the 2 NADPH

consumed in the reaction.
TV = total volume of the reaction in ml (1 ml for cuvettes)
V = volume of enzyme used in the assay (ml)
0.6 = enzyme concentration in mg-protein (mgP0/ml (0.55-0.65 mgP/ml)
LP = light path in cm (1 for cuvettes).

**Example 2**

The following table provides IC50 values for particular atorvastatin compounds of the present invention.

| Compound Structure | $IC_{50}$ (nM) |
|---|---|
| 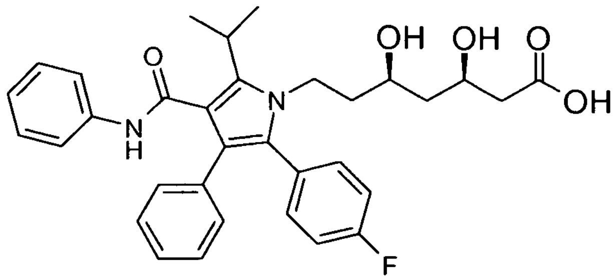 | 7 |
| 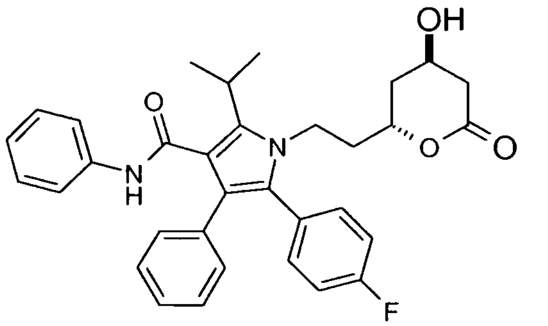 | 3 |
| 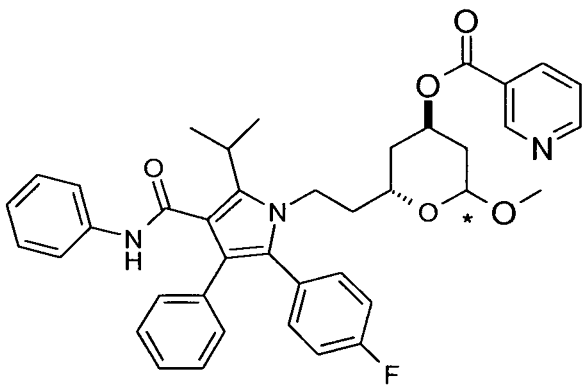 | <1 |
| 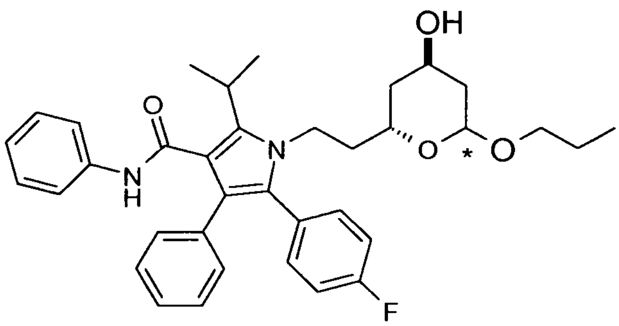 | <1 |

(continued)

| Compound Structure | IC50 (nM) |
|---|---|
| 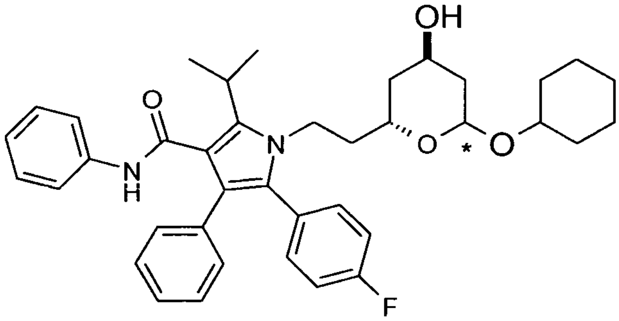 | <1 |
| 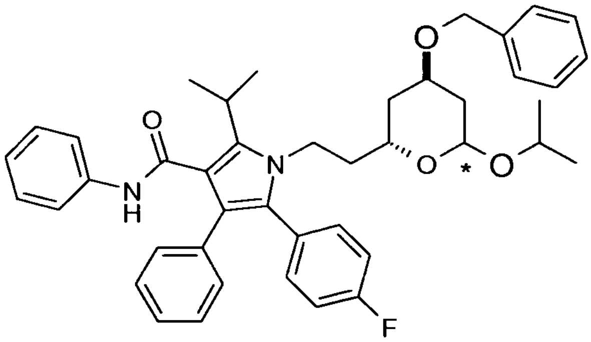 | 4 |
| 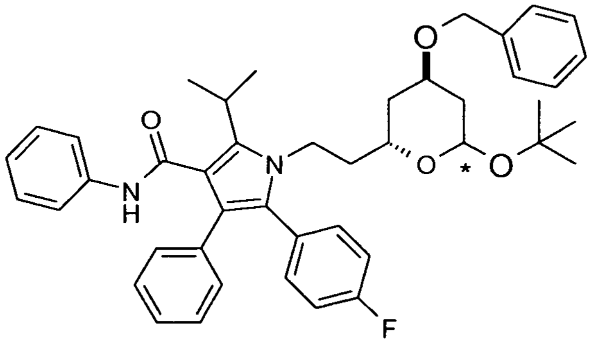 | 3 |
| 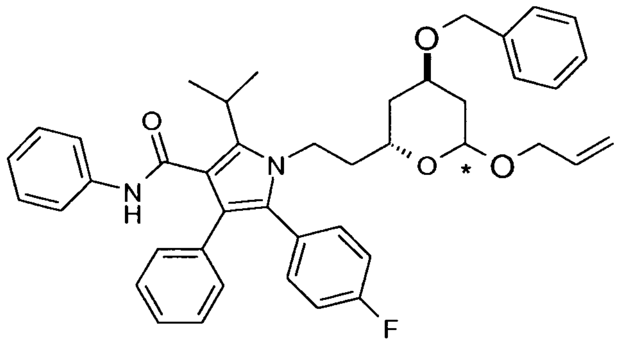 | 1 |

(continued)

| Compound Structure | $IC_{50}$ (nM) |
|---|---|
| 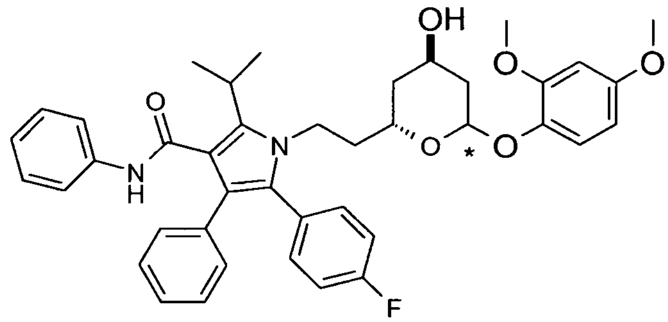 | <1 |

## Claims

1. A compound of Formula I and pharmaceutically acceptable salts and solvates thereof:

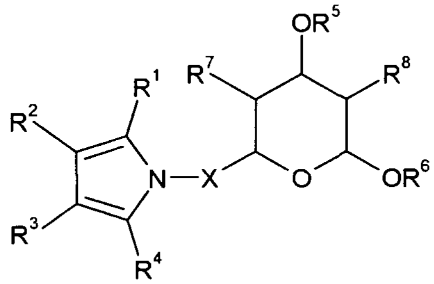

(I)

for use in treating a condition treatable by the inhibition of the enzyme 3-hydroxy-3-methylglutaryl-coenzyme A reductase (HMG-CoA reductase), wherein the condition treatable by the inhibition of HMG-CoA reductase is selected from the group consisting of: hypercholesterolemia, atherosclerosis, hyperlipidemia, cardiovascular disease, coronary heart disease, myocardial infarction, stroke, peripheral artery disease, inflammation, dementia, cancer, nuclear cataracts, diabetes and hypertension.
further wherein:

$R^1$, $R^4$ and one of $R^2$ and $R^3$ are independently selected from the group consisting of: hydrogen, halo, $C_{1-6}$ alkyl, $C_{2-6}$ alkenyl, $C_{3-6}$ cycloalkyl, aryl, $C_{1-4}$ alkyl aryl, heterocyclyl, and $C_{1-4}$ alkyl heteroaryl;
the other of $R^2$ and $R^3$ is $-CONR^9R^{10}$ where $R^9$ and $R^{10}$ are independently selected from the group consisting of: hydrogen, $C_{1-6}$alkyl, $C_{2-6}$ alkenyl, aryl, $C_{1-4}$ alkyl aryl, heteroaryl, $C_{1-4}$ heteroaryl;
$R^5$ and $R^6$ are independently selected from the group consisting of: hydrogen, $C_{1-6}$ alkyl, $C_{1-6}$ haloalkyl, $C_{2-6}$ alkenyl, $C_{3-6}$ cycloalkyl, aryl, $C_{1-6}$ alkyl aryl, $C_{1-6}$ alkanoyl aryl, heteroaryl, $C_{1-6}$ alkanoyl heteroaryl, and $C_{1-6}$ alkyl heteroaryl; provided always that both $R^5$ and $R^6$ are not hydrogen;
$R^7$ and $R^8$ are independently selected from the group consisting of: H, $C_{1-4}$ alkyl and halo;
X is $-(CR^aR^b)_m(CR^a{=}CR^b)_n(CR^aR^b)_o$ where $R^a$ and $R^b$ are independently selected from the group consisting of: H, methyl, ethyl and halo and m, n, and o are independently 0, 1, 2, or 3 provided that m + n + o is not more than 3; and wherein
each of the above groups $R^1$ to $R^{10}$ may, where chemically possible, be independently optionally substituted by from 1 to 5 groups chosen independently at each occurrence from the groups consisting of: halo, $C_{1-3}$ alkyl, halo $C_{1-3}$ alkyl, $C_{1-3}$ alkoxy, $C_{1-3}$ haloalkoxy, hydroxy, and cyano.

2. A compound for use according to claim 1, wherein $R^1$ is $C_{1-6}$ alkyl.

3. A compound for use according to claim 1 or claim 2, wherein $R^2$ is $-CONR^9R^{10}$ where $R^9$ is H and $R^{10}$ is aryl.

4. A compound for use according to any of claims 1 to 3, wherein $R^3$ is aryl.

**5.** A compound for use according to any preceding claim, wherein $R^4$ is optionally substituted aryl.

**6.** A compound for use according to any preceding claim, wherein $R^1$ is i-propyl, $R^2$ is -CONHPh, $R^3$ is phenyl and $R^4$ 4-fluorophenyl.

**7.** A compound for use according to any preceding claim, wherein $R^5$ is selected from the group consisting of: hydrogen, aryl, $C_{1-6}$ alkyl aryl, $C_{1-6}$ alkanoyl aryl, heteroaryl, $C_{1-6}$ alkanoyl heteroaryl, and $C_{1-6}$ alkyl heteroaryl.

**8.** A compound for use according to claim 7, wherein $R^5$ is hydrogen.

**9.** A compound for use according to claim 7, wherein $R^5$ is selected from the group consisting of: -$C_1$ alkyl-Ph, -$C_2$ alkyl-Ph, -$C_3$ alkyl-Ph, and -$C_4$ alkyl-Ph; optionally wherein $R^5$ is benzyl.

**10.** A compound for use according to claim 7, wherein $R^5$ is $C_{1-6}$alkanoyl pyridine; optionally wherein $R^5$ is 3-methanoyl pyridine.

**11.** A compound for use according to any preceding claim, wherein $R^6$ is selected from the group consisting of: hydrogen, $C_{1-6}$ alkyl, $C_{1-6}$ haloalkyl, $C_{2-6}$ alkenyl, $C_{3-6}$ cycloalkyl, optionally substituted aryl and $C_{1-6}$ alkyl aryl.

**12.** A compound for use according to claim 11, wherein $R^6$ is selected from the group consisting of: $C_{1-6}$ alkyl, $C_{3-6}$ cycloalkyl, $C_{2-6}$ alkenyl and optionally substituted aryl; optionally wherein $R^6$ is selected from the group consisting of: methyl, ethyl, propyl, butyl, cyclohexyl and allyl.

**13.** A compound for use according to claim 11, wherein $R^6$ is optionally substituted aryl; optionally wherein $R^6$ is selected from the group consisting of: $C_{1-6}$ alkoxy substituted phenyl; optionally wherein $R^6$ is 2,4-dimethoxyphenyl.

**14.** A compound for use according to any of claims 1 to claim 6, wherein $R^5$ is hydrogen and $R^3$ is an $C_{1-6}$ alkyl, $C_{3-6}$ cycloalkyl or an optionally substituted aryl.

**15.** A compound for use according to any of claims 1 to claim 6, wherein $R^5$ is an optionally substituted benzyl and $R^6$ is an optionally substituted $C_{1-6}$alkyl or an optionally substituted $C_{2-6}$ alkenyl.

**16.** A compound for use according to any of claims 1 to claim 6, wherein $R^5$ is a $C_{1-6}$ alkanoyl heteroaryl and $R^6$ is an optionally substituted $C_{1-6}$alkyl.

**17.** A compound for use according to any preceding claim wherein $R^7$ is H and $R^3$ is H.

**18.** A compound for use according to any preceding claim, wherein each $R^a$ is H, each $R^b$ is H and m = 2, n = 0 and o = 0.

**19.** A compound for use according to claim 1 which has a structure selected from:

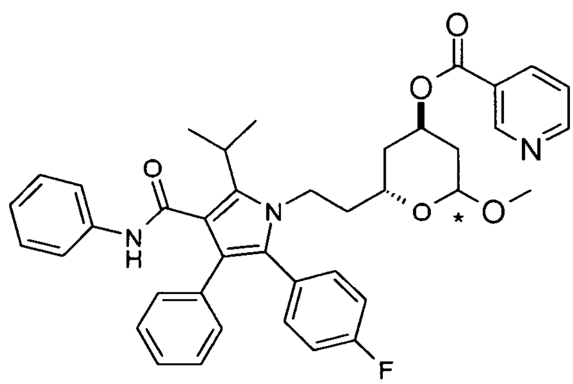

;

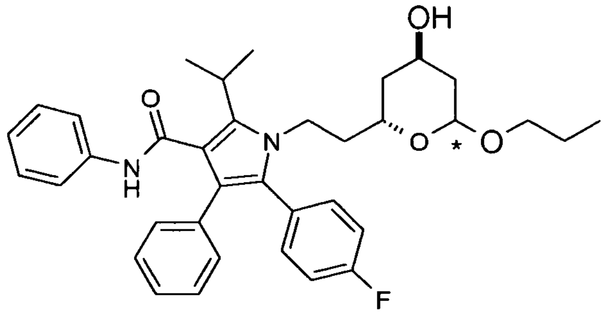

;

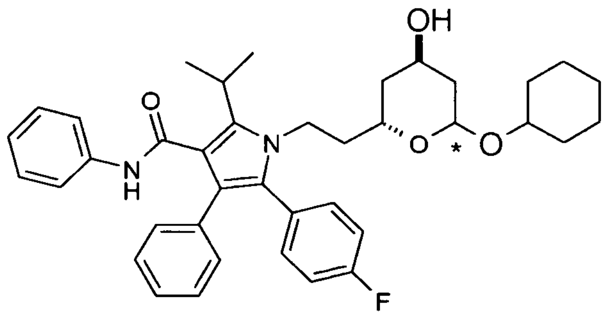

;

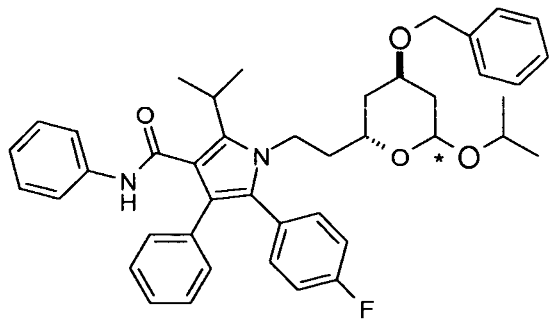

;

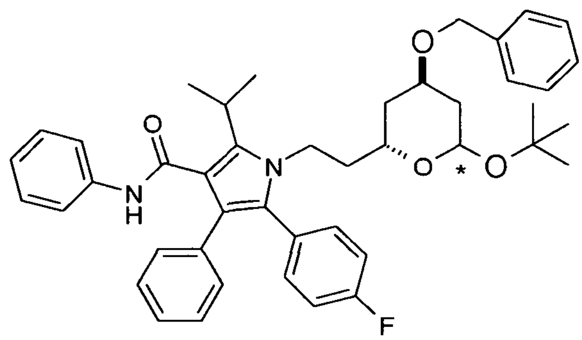

;

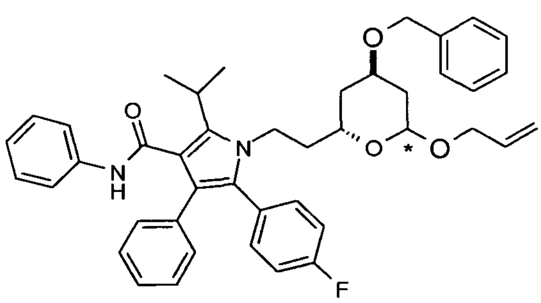

;

and

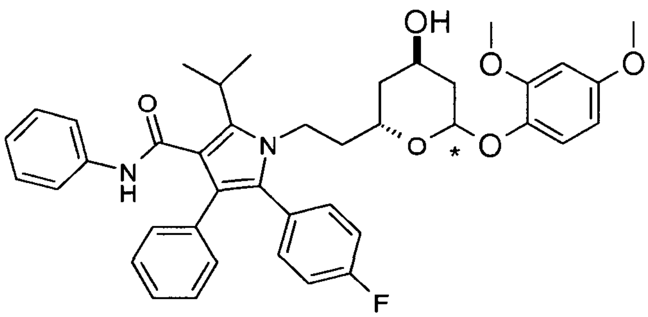

.

**Patentansprüche**

1. Verbindung der Formel I und pharmazeutisch unbedenkliche Salze und Solvate davon:

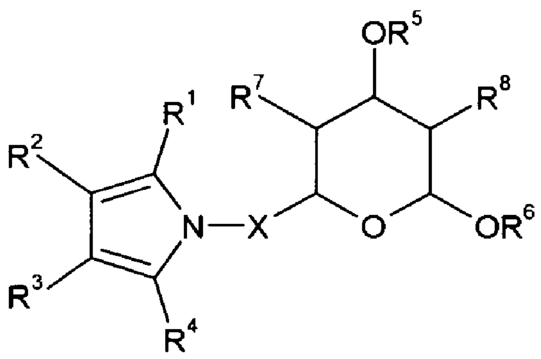

(I)

zur Verwendung bei der Behandlung eines Leidens, das durch die Hemmung des Enzyms 3-Hydroxy-3-methylglutaryl-Coenzym-A-Reduktase (HMG-CoA-Reduktase) behandelbar ist, wobei das Leiden, das durch die Hemmung von HMG-CoA-Reduktase behandelbar ist, aus der Gruppe bestehend aus folgenden ausgewählt ist: Hypercholesterinämie, Atherosklerose, Hyperlipidämie, Herz-Kreislauf-Erkrankung, koronare Herzkrankheit, Herzinfarkt, Schlaganfall, periphere arterielle Erkrankung, Entzündung, Demenz, Krebs, Kernstarerkrankungen, Diabetes und Hypertonie,
weiterhin wobei:

wobei $R^1$, $R^4$ und einer von $R^2$ und $R^3$ unabhängig aus der Gruppe bestehend aus folgenden ausgewählt sind: Wasserstoff, Halogen, $C_{1-6}$-Alkyl, $C_{2-6}$-Alkenyl, $C_{3-6}$-Cycloalkyl, Aryl, $C_{1-4}$-Alkylaryl, Heterocyclyl und $C_{1-4}$-Alkylheteroaryl;
der anderevon $R^2$ und $R^3$ -$CONR^9R^{10}$ ist, wobei $R^9$ und $R^{10}$ unabhängig aus der Gruppe bestehend aus folgenden ausgewählt sind: Wasserstoff, $C_{1-6}$-Alkyl, $C_{2-6}$-Alkenyl, Aryl, $C_{1-4}$-Alkylaryl, Heteroaryl und $C_{1-4}$-He-

teroaryl;
$R^5$ und $R^6$ unabhängig aus der Gruppe bestehend aus folgenden ausgewählt sind: Wasserstoff, $C_{1-6}$-Alkyl, $C_{1-6}$-Halogenalkyl, $C_{2-6}$-Alkenyl, $C_{3-6}$-Cycloalkyl,Aryl, $C_{1-6}$-Alkylaryl, $C_{1-6}$-Alkanoylaryl, Heteroaryl, $C_{1-6}$-Alkanoylheteroaryl und $C_{1-6}$-Alkylheteroaryl; immer vorausgesetzt, dass sowohl $R^5$ als auch $R^6$ nicht Wasserstoff sind;
$R^7$ und $R^8$ unabhängig aus der Gruppe bestehend aus folgenden ausgewählt sind: H, $C_{1-4}$-Alkyl und Halogen;
X $-(CR^aR^b)_m(CR^a{=}R^b)_n(CR^aR^b)_o$ ist, wobei $R^a$ und $R^b$ unabhängig aus der Gruppe bestehend aus folgenden ausgewählt sind: H, Methyl, Ethyl und Halogen, und m, n und o unabhängig 0, 1, 2 oder 3 sind, vorausgesetzt, dass m + n + o nicht mehr als 3 ist; und wobei
jede der obigen Gruppen $R^1$ bis $R^{10}$, soweit chemisch möglich, unabhängig gegebenenfalls durch 1 bis 5 Gruppen substituiert sein kann, die bei jedem Vorkommen unabhängig aus den Gruppen bestehend aus folgenden ausgewählt sind: Halogen, $C_{1-3}$-Alkyl, Halogen-$C_{1-3}$-alkyl, $C_{1-3}$-Alkoxy, $C_{1-3}$-Halogenalkoxy, Hydroxy und Cyano.

**2.** Verbindung zur Verwendung nach Anspruch 1, wobei $R^1$ $C_{1-6}$-Alkyl ist.

**3.** Verbindung zur Verwendung nach Anspruch 1 oder 2, wobei $R^2$ $-CONR^9R^{10}$ ist, wobei $R^9$ H ist und $R^{10}$ Aryl ist.

**4.** Verbindung zur Verwendung nach einem der Ansprüche 1 bis 3, wobei $R^3$ Aryl ist.

**5.** Verbindung zur Verwendung nach einem vorhergehendenAnspruch, wobei $R^4$ gegebenenfalls substituiertes Aryl ist.

**6.** Verbindung zur Verwendung nach einem vorhergehendenAnspruch, wobei $R^1$ Isopropyl ist, $R^2$-CONHPh ist, $R^3$ Phenyl ist und $R^4$ 4-Fluorphenyl ist.

**7.** Verbindung zur Verwendung nach einem vorhergehendenAnspruch, wobei $R^5$ aus der Gruppe bestehend aus folgenden ausgewählt ist: Wasserstoff, Aryl, $C_{1-6}$-Alkylaryl, $C_{1-6}$-Alkanoylaryl, Heteroaryl, $C_{1-6}$-Alkanoylheteroaryl und $C_{1-6}$-Alkylheteroaryl.

**8.** Verbindung zur Verwendung nach Anspruch 7, wobei $R^5$ Wasserstoff ist.

**9.** Verbindung zur Verwendung nach Anspruch 7, wobei $R^5$ aus der Gruppe bestehend aus folgenden ausgewählt ist: $-C_1$-Alkyl-Ph, $-C_2$-Alkyl-Ph, $-C_3$-Alkyl-Ph und $-C_4$-Alkyl-Ph; gegebenenfalls wobei $R^5$ Benzyl ist.

**10.** Verbindung zur Verwendung nach Anspruch 7, wobei $R^5$ $C_{1-6}$-Alkanoylpyridin ist; gegebenenfalls wobei $R^5$ 3-Methanoylpyridin ist.

**11.** Verbindung zur Verwendung nach einem vorhergehendenAnspruch, wobei $R^6$ aus der Gruppe bestehend aus folgenden ausgewählt ist: Wasserstoff, $C_{1-6}$-Alkyl, $C_{1-6}$-Halogenalkyl, $C_{2-6}$-Alkenyl, $C_{3-6}$-Cycloalkyl, gegebenenfalls substituiertes Aryl und $C_{1-6}$-Alkylaryl.

**12.** Verbindung zur Verwendung nach Anspruch 11, wobei $R^6$ aus der Gruppe bestehend aus folgenden ausgewählt ist: $C_{1-6}$-Alkyl, $C_{3-6}$-Cycloalkyl, $C_{2-6}$-Alkenyl und gegebenenfalls substituiertes Aryl; gegebenenfalls wobei $R^6$ aus der Gruppe bestehend aus folgenden ausgewählt ist: Methyl, Ethyl, Propyl, Butyl, Cyclohexyl und Allyl.

**13.** Verbindung zur Verwendung nach Anspruch 11, wobei $R^6$ gegebenenfalls substituiertes Aryl ist; gegebenenfalls wobei $R^6$ aus der Gruppe bestehend aus folgender ausgewählt ist: $C_{1-6}$-alkoxysubstituiertes Phenyl; gegebenenfalls wobei $R^6$ 2,4-Dimethoxyphenyl ist.

**14.** Verbindung zur Verwendung nach einem der Ansprüche 1 bis 6, wobei $R^5$ Wasserstoff ist und $R^6$ ein $C_{1-6}$-Alkyl, $C_{3-6}$-Cycloalkyl oder ein gegebenenfalls substituiertes Aryl ist.

**15.** Verbindung zur Verwendung nach einem der Ansprüche 1 bis 6, wobei $R^5$ ein gegebenenfalls substituiertes Benzyl ist und $R^6$ ein gegebenenfalls substituiertes $C_{1-6}$-Alkyl oder ein gegebenenfalls substituiertes $C_{2-6}$-Alkenyl ist.

**16.** Verbindung zur Verwendung nach einem der Ansprüche 1 bis 6, wobei $R^5$ ein $C_{1-6}$-Alkanoylheteroaryl ist und $R^6$ ein gegebenenfalls substituiertes $C_{1-6}$-Alkyl ist.

**17.** Verbindung zur Verwendung nach einem vorhergehendenAnspruch, wobei $R^7$ H ist und $R^8$ H ist.

**18.** Verbindung zur Verwendung nach einem vorhergehendenAnspruch, wobei jedes $R^a$ H ist, jedes $R^b$ H ist und m = 2, n = 0 und o = 0.

**19.** Verbindung zur Verwendung nach Anspruch 1, die eine Struktur aufweist, die aus folgenden ausgewählt ist:

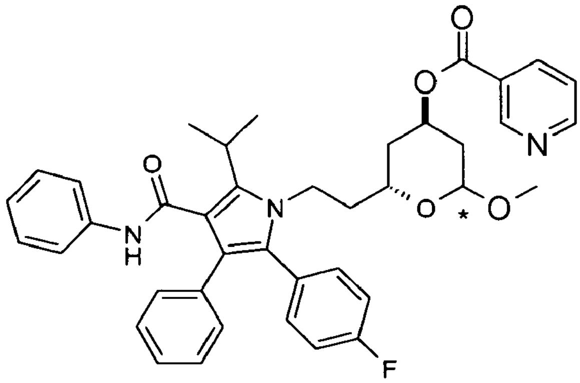

;

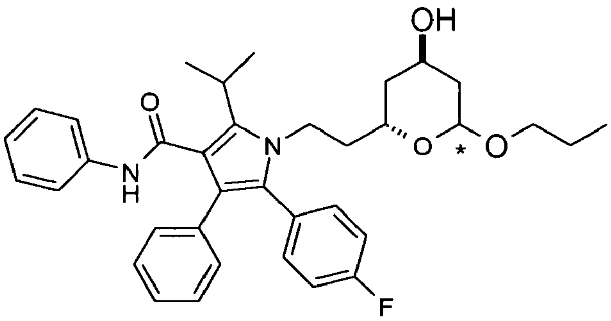

;

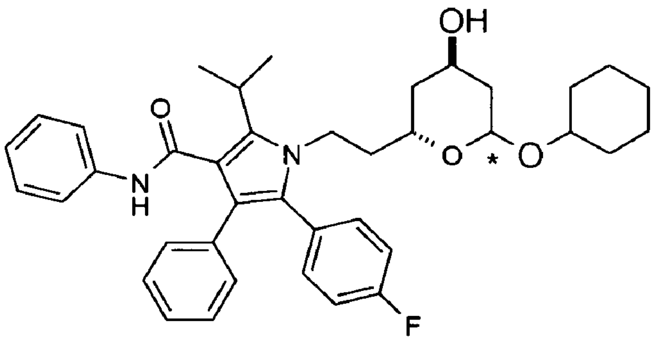

;

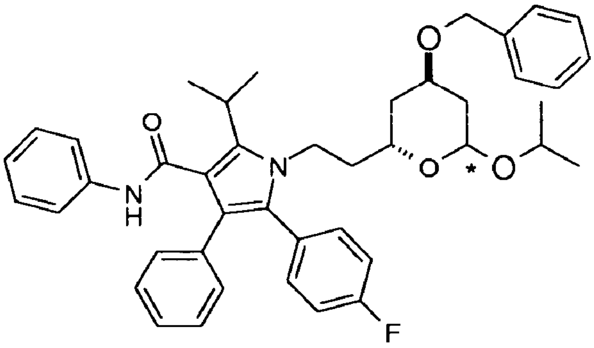

;

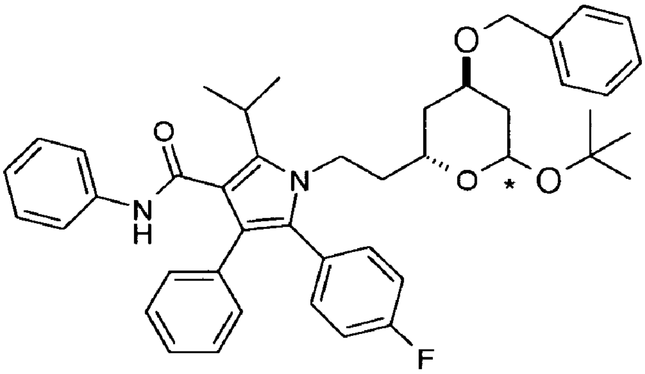

;

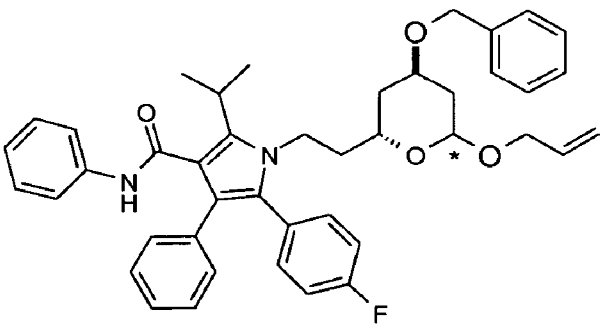

und

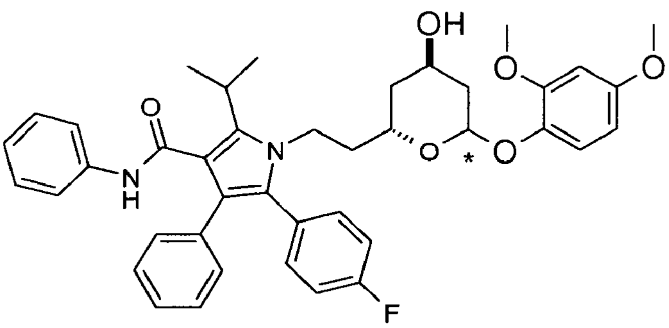

.

**Revendications**

1. Composé de Formule I et sels et solvates pharmaceutiquement acceptables de celui-ci :

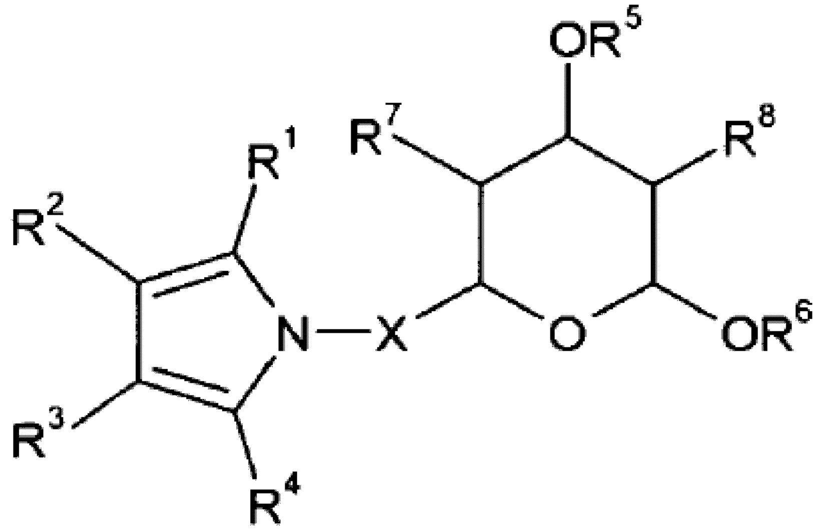

(I)

destiné à être utilisé dans le traitement d'une affection pouvant être traitée par l'inhibition de l'enzyme 3-hydroxy-3-méthylglutaryl-coenzyme A réductase (HMG-CoA réductase), dans lequel l'affection pouvant être traitée par l'inhibition de la HMG-CoA réductase est sélectionnée parmi le groupe constitué de : l'hypercholestérolémie, l'athérosclérose, l'hyperlipidémie, une maladie cardiovasculaire, une maladie coronarienne, un infarctus du myocarde, un accident vasculaire cérébral, une maladie des artères périphériques, une inflammation, la démence, un cancer, des cataractes nucléaires, le diabète et l'hypertension ; dans lequel en outre :

$R^1$, $R^4$ et un de $R^2$ et $R^3$ sont indépendamment sélectionnés parmi le groupe constitué de : hydrogène, halogéno, alkyle en $C_{1-6}$, alkényle en $C_{2-6}$, cycloalkyle en $C_{3-6}$, aryle, alkylaryle en $C_{1-4}$, hétérocyclyle et alkylhétéroaryle en $C_{1-4}$ ;
l'autre de $R^2$ et $R^3$ est $-CONR^9R^{10}$, où $R^9$ et $R^{10}$ sont indépendamment sélectionnés parmi le groupe constitué de : hydrogène, alkyle en $C_{1-6}$, alkényle en $C_{2-6}$, aryle, alkylaryle en $C_{1-4}$, hétéroaryle et hétéroaryle en $C_{1-4}$ ;
$R^5$ et $R^6$ sont indépendamment sélectionnés parmi le groupe constitué de : hydrogène, alkyle en $C_{1-6}$, halogénoalkyle en $C_{1-6}$, alkényle en $C_{2-6}$, cycloalkyleen $C_{3-6}$, aryle, alkylaryle en $C_{1-6}$, alkanoylaryle en $C_{1-6}$, hétéroaryle, alkanoylhétéroaryle en $C_{1-6}$ et alkylhétéroaryle en $C_{1-6}$ ; à condition toujours que $R^5$ et $R^6$ ne soient pas tous deux hydrogène ;
$R^7$ et $R^8$ sont indépendamment sélectionnés parmi le groupe constitué de : H, alkyle en $C_{1-4}$ et halogéno ;
X est $-(CR^aR^b)_m(CR^a=CR^b)_n(CR^aR^b)_o$, où $R^a$ et $R^b$ sont indépendamment sélectionnés parmi le groupe constitué de : H, méthyle, éthyle et halogéno, et m, n et o sont indépendamment 0, 1, 2 ou 3 à condition que m + n + o ne fasse pas plus de 3 ; et dans lequel
chacun des groupes $R^1$ à $R^{10}$ ci-dessus peut, lorsque c'est chimiquement possible, être indépendamment substitué en option par 1 à 5 groupes choisis indépendamment à chaque occurrence parmi les groupes constitués de : halogéno, alkyle en $C_{1-3}$, halogéno-$C_{1-3}$-alkyle, alkoxy en $C_{1-3}$, halogénoalkoxy en $C_{1-3}$, hydroxy et cyano.

**2.** Composé destiné à être utilisé selon la revendication1, dans lequel $R^1$ est alkyle en $C_{1-6}$.

**3.** Composé destiné à être utilisé selon la revendication1 ou la revendication2, dans lequel $R^2$ est $-CONR^9R^{10}$, où $R^9$ est H et $R^{10}$ est aryle.

**4.** Composé destiné à être utilisé selon l'une quelconque des revendications 1 à 3, dans lequel $R^3$ est aryle.

**5.** Composé destiné à être utilisé selon l'une quelconque des revendications précédentes, dans lequel $R^4$ est aryle substitué en option.

**6.** Composé destiné à être utilisé selon l'une quelconque des revendications précédentes, dans lequel $R^1$ est i-propyle, $R^2$ est -CONHPh, $R^3$ est phényle et $R^4$ 4-fluorophényle.

**7.** Composé destiné à être utilisé selon l'une quelconque des revendications précédentes, dans lequel $R^5$ est sélec-

tionné parmi le groupe constitué de : hydrogène, aryle, alkylaryle en $C_{1-6}$, alkanoylaryle en $C_{1-6}$, hétéroaryle, alkanoylhétéroaryle en $C_{1-6}$ et alkylhétéroaryle en $C_{1-6}$.

**8.** Composé destiné à être utilisé selon la revendication7, dans lequel $R^5$ est hydrogène.

**9.** Composé destiné à être utilisé selon la revendication7, dans lequel $R^5$ est sélectionné parmi le groupe constitué de : $-C_1$-alkyle-Ph, $-C_2$-alkyle-Ph, $-C_3$-alkyle-Ph et $-C_4$-alkyle-Ph ; en option dans lequel $R^5$ est benzyle.

**10.** Composé destiné à être utilisé selon la revendication7, dans lequel $R^5$ est alkanoylpyridine en $C_{1-6}$ ; en option dans lequel $R^5$ est 3-méthanoylpyridine.

**11.** Composé destiné à être utilisé selon l'une quelconque des revendications précédentes, dans lequel $R^6$ est sélectionné parmi le groupe constitué de : hydrogène, alkyle en $C_{1-6}$, halogénoalkyle en $C_{1-6}$, alkényle en $C_{2-6}$, cycloalkyleen $C_{3-6}$, aryle substitué en option et alkylaryle en $C_{1-6}$.

**12.** Composé destiné à être utilisé selon la revendication 11, dans lequel $R^6$ est sélectionné parmi le groupe constitué de : alkyle en $C_{1-6}$, cycloalkyle en $C_{3-6}$, alkényle en $C_{2-6}$ et aryle substitué en option ; en option dans lequel $R^6$ est sélectionné parmi le groupe constitué de : méthyle, éthyle, propyle, butyle, cyclohexyle et allyle.

**13.** Composé destiné à être utilisé selon la revendication 11, dans lequel $R^6$ est aryle substitué en option ; en option dans lequel $R^6$ est sélectionné parmi le groupe constitué de : phényle substitué par alkoxy en $C_{1-6}$ ; en option dans lequel $R^6$ est 2,4-diméthoxyphényle.

**14.** Composé destiné à être utilisé selon l'une quelconque des revendications 1 à la revendication6, dans lequel $R^5$ est hydrogène et $R^6$ est un alkyle en $C_{1-6}$, cycloalkyle en $C_{3-6}$ ou un aryle substitué en option.

**15.** Composé destiné à être utilisé selon l'une quelconque des revendications 1 à la revendication6, dans lequel $R^5$ est un benzyle substitué en option et $R^6$ est un alkyle en $C_{1-6}$ substitué en option ou un alkényle en $C_{2-6}$ substitué en option.

**16.** Composé destiné à être utilisé selon l'une quelconque des revendications 1 à la revendication6, dans lequel $R^5$ est un alkanoylhétéroaryle en $C_{1-6}$ et $R^6$ est un alkyle en $C_{1-6}$ substitué en option.

**17.** Composé destiné à être utilisé selon l'une quelconque des revendications précédentes, dans lequel $R^7$ est H et $R^8$ est H.

**18.** Composé destiné à être utilisé selon l'une quelconque des revendications précédentes, dans lequel chaque $R^a$ est H, chaque $R^b$ est H et m = 2, n = 0 et o = 0.

**19.** Composé destiné à être utilisé selon la revendication 1 qui possède une structure sélectionnée parmi :

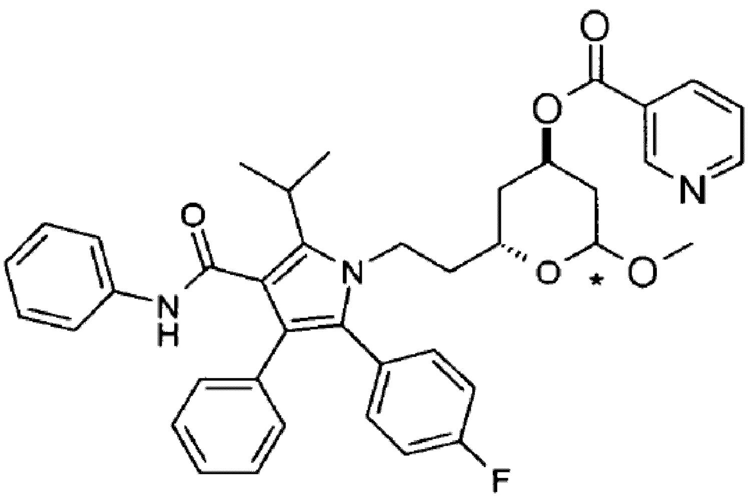

;

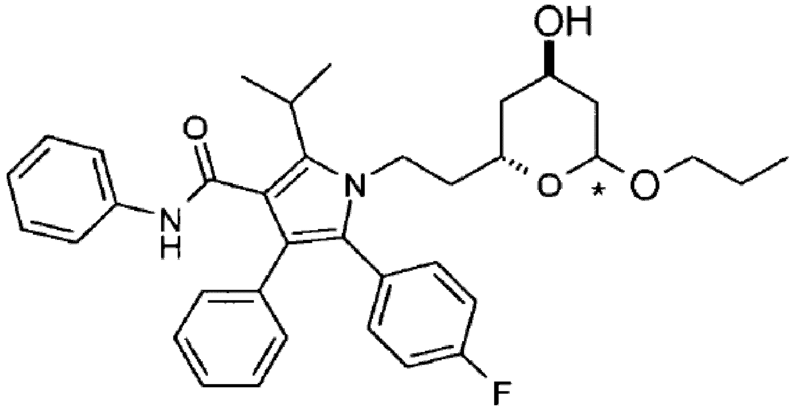

;

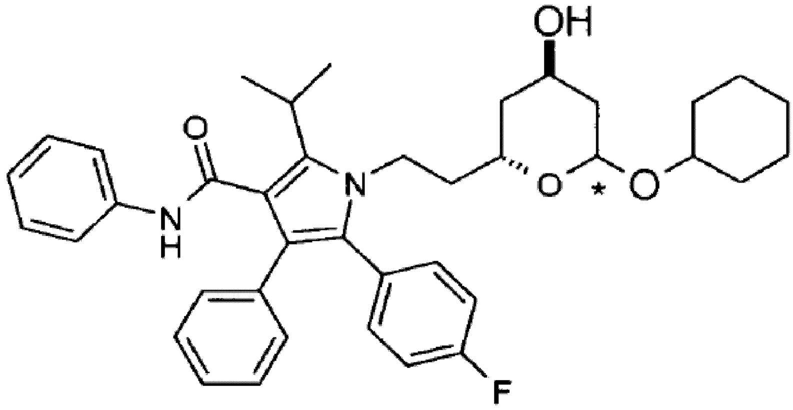

;

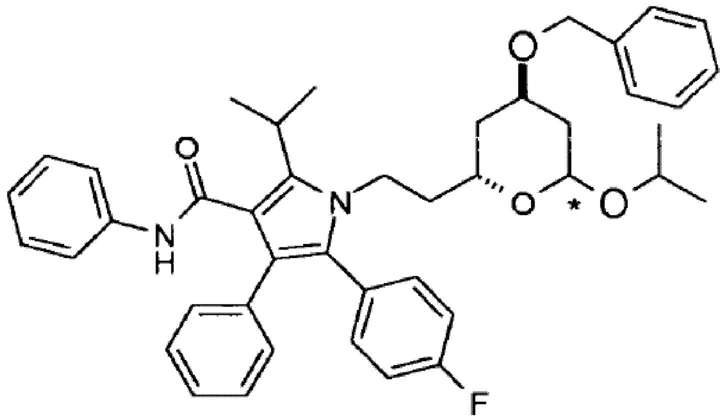

;

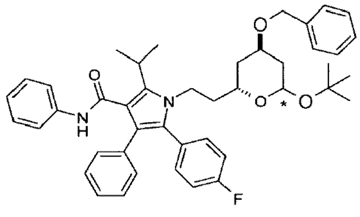

;

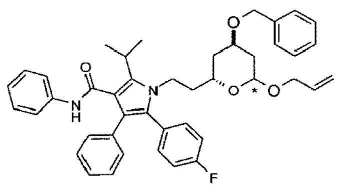

;

et

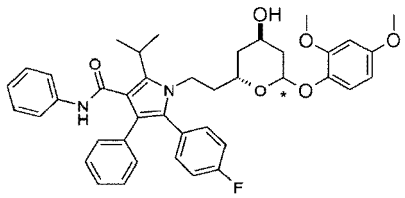

.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4681893 A
- EP 0409281 A
- WO 2005012246 A


### Non-patent literature cited in the description


- **BAKER ; TAMOPOLSKY.** *Clin Invest Med,* 2001, vol. 24 (5), 258-72
- *Trends in Pharmacological Sciences,* 01 January 1998, vol. 19 (1), 26-37
- **STAHL ; WERMUTH.** Handbook of Pharmaceutical Salts: Properties, Selection, and Use. Wiley-VCH, 2002
- **HALEBLIAN.** *J Pharm Sci,* August 1975, vol. 64 (8), 1269-1288